# EUROPEAN PATENT APPLICATION

(11) **EP 4 166 651 A1**
(43) Date of publication of application: **19.04.2023**
(21) Application number: 21203187.6
(22) Date of filing: 18.10.2021
(51) Int. Cl.: C12N 5/00

(54) **METHODS AND COMPOSITION TO INCREASE CELLULAR GLUTATHIONE LEVEL**

(71) Applicant: Evonik Operations GmbH, 45128 Essen (DE)
(72) Inventor: BENEDIKT, Anne, 60431 Frankfurt (DE); JOST, Christina, 64665 Alsbach-Hähnlein (DE); SCHILLING, Martin, 53121 Bonn (DE); KEßLER, Christian, 63741 Aschaffenburg (DE); GOMEZ, Mario, 64297 Darmstadt (DE); RANDL Stefan, 60599 Frankfurt (DE)
(74) Representative: Evonik Patent Association

(57) **Abstract**

The present invention relates to a method for increasing intracellular level of glutathione in cultured cells, wherein the cultured cells are growing in a specific cell culture medium, and the use of such cell culture medium for the increase of intracellular glutathione level in culture cells.

## Description

### FIELD OF THE INVENTION

The present invention relates to a method for increasing intracellular level of glutathione in cultured cells, wherein the cultured cells are growing in a specific cell culture medium, and the use of such cell culture medium for the increase of intracellular glutathione level in culture cells. Moreover, the present invention relates to biotechnological production processes. More specifically, the present invention relates to improved culture media for use in biotechnological production processes, processes employing such improved media, and to products obtained from the processes using the improved culture media.

### BACKGROUND OF THE INVENTION

Glutathione is an important redox regulator in all animal and mammalian cells and essential to fight oxidative stress. Cells in culture require optimized nutrient supply to enable them to synthesize sufficient glutathione. This is especially true for modern, highly intensified cell culture processes that are used in biopharmaceutical production, e.g. in the production of monoclonal antibodies in Chinese hamster ovary (CHO) cells at very high cell densities (Chevallier et al., Biotechnology and Bioengineering. 2020;117:3448-3458). It is also relevant for the cultivation of therapeutic cells like mesenchymal stromal cells (Jeong et al., International Journal of Stem Cells Vol. 12, No. 2, 2019).

The amino acid L-cysteine is an important glutathione precursor but participates in redox reactions with metal ions and contributes to ROS formation. In these reactions, L-cysteine is oxidized to the disulfide form L-cystine, which has a very low solubility. Therefore, there remains a need for other, more soluble and less reactive ingredients that can increase intracellular glutathione levels (Graham et al., Biotechnology and Bioengineering. 2019;116:3446-3456). Many alternatives to increase glutathione supply have been investigated, as summarized in Chevallier et al. (Biotechnology and Bioengineering. 2020;117:1172-1186) or WO2017/016631 A1, which describes a method for increasing the glutathione level in cells by use of sulfocysteine and derivatives. EP3492582A1 relates to methods for reducing the heterogeneity of a population of recombinant proteins produced in cell culture, said methods comprising growing host cells producing a recombinant protein in a cell culture medium wherein the cell culture medium comprises one or more cysteine/cystine analogs. In this publication, negative effects of S-sulfocysteine on growth and product formation are described, which shows that there remains a need for highly soluble and biocompatible Cys-sources that contribute to increased intracellular glutathione levels.

### SUMMARY OF THE INVENTION

The above shortcomings are addressed by the present invention. The invention is defined by the terms of the appended independent claims. Preferred embodiments of the invention are defined by the dependent claims.

Surprisingly it was found that the addition of dipeptides to cell culture media increases the intracellular glutathione level. Especially the addition of Cys-dipeptides resulted in an increase. Mixtures of L-cysteine with Cys-dipeptides were even more effective.

The present invention relates to a method for increasing intracellular level of glutathione in cultured cells, wherein the cultured cells are growing in a cell culture medium, wherein the cell culture medium comprises at least one oligopeptide of 2-10 amino acids in length, said amino acids being natural amino acids and at least one of said amino acid being cysteine (Cys).

The invention further relates to the use of a cell culture medium for increasing intracellular level of glutathione in cultured cells, preferably as an aqueous stock or feed solution, wherein the cell culture medium comprises at least one oligopeptide of 2-10 amino acids in length, said amino acids being natural amino acids and at least one of said amino acid being cysteine (Cys) and free cysteine.

Another aspect of the invention relates to a method of manufacturing a cell culture product comprising the steps of (i) providing a cell capable of producing said cell culture product; (ii) contacting said cell with a culture medium according to the invention; and (iii) obtaining said cell culture product from said culture medium or from said cell.

Preferred embodiments of the invention are described in further detail in the following detailed description of the invention.

### DETAILED DESCRIPTION OF THE INVENTION

In the context of the present invention, the expression "natural amino acids" shall be understood to include both the L-form and the D-form of the above listed 20 amino acids. The L-form, however, is preferred. In one embodiment, the term "amino acid" also includes analogues or derivatives of those amino acids.

A "free amino acid", according to the invention, for instance "free" cysteine, is understood as being an amino acid having its amino and its (alpha-) carboxylic functional group in free form, i.e., not covalently bound to other molecules, e.g., an amino acid not forming a peptide bond. Free amino acids may also be present as salts or in hydrate form. When referring to an amino acid as a part of, or in, a dipeptide, this shall be understood as referring to that part of the respective dipeptide structure derived from the respective amino acid, according to the known mechanisms of biochemistry and peptide biosynthesis.

The present invention generally relates to a composition comprising at least one oligopeptide with one amino acid being cysteine (Cys) and (as a cysteine source) free cysteine. Optionally free cysteine and free cystine (Cys-Cys) may be comprised. The terms free cysteine and free cystine (Cys-Cys) shall include the salts or hydrate forms of cysteine and cystine.

Preferably the oligopeptide is a dipeptide.

A "peptide" shall be understood as being a molecule comprising at least two amino acids covalently coupled to each other by alpha-peptide bonds (R¹-CO-NH-R²).

A "polypeptide" shall be understood as being a molecule comprising more than twenty amino acids covalently coupled to each other by peptide bonds (R¹-CO-NH-R²).

An "oligopeptide" shall be understood as being a molecule comprising less than twenty, preferably two to ten amino acids covalently coupled to each other solely by alpha-peptide-bonds (R¹-CO-NH-R²). Glutathione, a Glu-Cys-Gly tripeptide, which contains a gamma-peptide-bond and an alpha-peptide-bond is therefore excluded.

A "dipeptide" shall be understood as being a molecule comprising two amino acids covalently coupled to each other by an alpha-peptide-bond (R¹-CO-NH-R²).

The expression "Xxx", when used herein in connection with an amino acid, shall be understood as referring to any natural amino acid as defined in the following.

An "amino acid", in the context of the present invention, shall be understood as being a molecule comprising an amino functional group (-NH₂) and a carboxylic acid functional group (-COOH), along with a side-chain specific to the respective amino acid. In the context of the present invention, both alpha- and beta-amino acids are included. Preferred amino acids of the invention are alpha-amino acids, in particular the 20 "natural amino" acids including cystine as follows:
- Alanine: (Ala / A)
- Arginine: (Arg / R)
- Asparagine: (Asn / N)
- Aspartic acid: (Asp / D)
- Cysteine: (Cys / C)
- Cystine: (Cyss/C2)
- Glutamic acid: (Glu / E)
- Glutamine: (Gln / Q)
- Glycine: (Gly / G)
- Histidine: (His / H)
- Isoleucine: (Ile / I)
- Leucine: (Leu / L)
- Lysine: (Lys / K)
- Methionine: (Met / M)
- Phenylalanine: (Phe / F)
- Proline: (Pro / P)
- Serine: (Ser / S)
- Threonine: (Thr / T)
- Tryptophan: (Trp / W)
- Tyrosine: (Tyr / Y)
- Valine: (Val/V)

In the context of the present invention, the expression "natural amino acids" shall be understood to include both the L-form and the D-form of the above listed 20 amino acids. The L-form, however, is preferred. In one embodiment, the term "amino acid" also includes analogues or derivatives of those amino acids.

A "free amino acid", according to the invention (for instance "free cysteine"), is understood as being an amino acid having its amino and its (alpha-) carboxylic functional group in free form, i.e., not covalently bound to other molecules, e.g., an amino acid not forming a peptide bond. Free amino acids may also be present as salts or in hydrate form. When referring to an amino acid as a part of, or in, a dipeptide, this shall be understood as referring to that part of the respective dipeptide structure derived from the respective amino acid, according to the known mechanisms of biochemistry and peptide biosynthesis.

The expression "N-acylated", with reference to a chemical compound, such as an amino acid, shall be understood as meaning that the N-acylated compound is modified by the addition of an acyl group to a nitrogen functional group of said compound. Preferably, the acyl group is added to the alpha-amino group of the amino acid.

In a preferred configuration of the present invention, the oligopeptide is a dipeptide.

In another preferred configuration, the composition further comprises free cysteine.

The compositions can be prepared by mixing defined molar ratios of Cys-oligopeptide or Cys-dipeptide with cysteine or by mixing defined molar ratios of Cys-dipeptide with cysteine and cystine. The preferred molar ratio of the oligopeptide bound cysteine to the free cysteine is 10 or lower, preferably 4 or lower, more preferably 2 or lower, more preferable 1 or lower, more preferable 0.5 or lower, most preferable 0.2 or lower. The mixtures can be in the form of solids (crystalline powders, agglomerates etc) or aqueous solutions. In the case of aqueous solutions, cysteine is added in a concentration of at least 1 mM, preferable at least 10 mM, more preferable at least 50 mM and most preferable at least 100 mM and the dipeptide is added at the appropriate molar ratio described above.

The invention thus also relates to compositions prepared by mixing at least one oligopeptide, preferably at least one dipeptide, with one amino acid being cysteine (Cys), and a cysteine source selected from free cysteine and optionally cystine (Cys-Cys). The invention thus relates to a culture medium comprising the composition.

In the context of this invention, Cys-peptides forming a disulfide bond via oxidized cysteine residues, shall be described by (Xxx-Cys)₂. The peptides may also be present as salts or in hydrate form. Such disulfide bond mediated dimers of Cys-dipeptides, for instance (Xxx-Cys)₂, are still considered as a dipeptides in the sense of the invention.

Preferably, the composition has a pH-value at 25 °C of at least 5 or preferred of at least 6.

In an advantageous configuration of the present invention, a molar ratio of the peptide-bound cysteine to free cysteine is between 0.1 and 10, preferably between 0.2 and 4 or lower, most preferable between 0.5 and 2 In a preferred embodiment, the oligo- or dipeptides are either in a reduced state (= free thiol) or oxidized state (= disulfide bonded), preferably in an oxidized state In an alternative embodiment, the composition comprises a mixed disulfide of the dipeptide and the cysteine source.

In a preferred embodiment of the present invention, the oligo- or dipeptide further comprises one or more natural amino acids with a solubility of at least > 10 g/l at a pH range between pH 6 and pH 9 and is preferably selected from glycine (Gly), alanine (Ala), serine (Ser), proline (Pro), aspartic acid (Asp), glutamic acid (Glu), lysine (Lys) or arginine (Arg).

It is preferred, when said oligopeptide is a dipeptide which is Xxx-Cys or Cys-Xxx, wherein Xxx is a natural amino acid. The natural amino acid Xxx is preferably selected from Ala and Lys. The dipeptide is preferably selected from Asp-Cys, Cys-Asp, Lys-Cys, Cys-Lys, Ala-Cys or Pro-Cys.

In a preferred configuration, said dipeptide is present in said culture medium at a concentration of at least 1 mM, preferably at least 10 mM, more preferably at least 50 mM, more preferably at least 100 mM. At such high concentrations, the composition according to the present invention provides the advantage that the cysteine-containing dipeptides stabilize cysteine against oxidative precipitation.

In a preferred configuration, the dipeptide is selected from Lys-Cys, Cys-Lys, Cys-Ala and Ala-Cys. It is most preferred to use Lys-Cys or Ala-Cys.

In preferred embodiments, the oligo- or dipeptide is not N-acylated. N-acylation is known to improve heat stability of certain dipeptide; however, it has been found that N-acylated dipeptides may also lead to inferior viable cell density and viability.

The present invention is also directed to a cosmetic product, a nutritional supplement or nutrient solution for clinical nutrition comprising the composition according to the present invention.

The cosmetic product may be a shampoo, conditioner, lotion, cream or other formulations used to treat skin or hair. Nutritional supplements may be in liquid form, such as syrups or shots, or in solid form, such as capsules, soft-gels, gummies. The compositions can also be part of nutrient solutions for clinical enteral or parenteral nutrition, e.g. part of an amino acid solution such as Aminoven (Fresenius Kabi).

Moreover, the present invention also refers to a cell or tissue culture medium.

Another subject of the present invention is directed to a cell or tissue culture medium comprising the composition according to the present invention, which further comprises at least one carbohydrate, at least one free amino acid, at least one inorganic salt, a buffering agent and/or at least one vitamin. In a particularly preferred embodiment, the culture medium comprises all of at least one carbohydrate, at least one free amino acid, at least one inorganic salt, a buffering agent and at least one vitamin.

In one embodiment of the invention, the culture medium does not contain a growth factor. In accordance with this embodiment, the oligo- or dipeptide of the invention may be used instead of a growth factor for promoting growth and/or proliferation of the cells in culture. In another embodiment of the invention, the culture medium does not contain any lipids.

According to another embodiment of the invention, the culture medium is in liquid form, in form of a gel, a powder, a granulate, a pellet or in form of a tablet.

In preferred embodiments, the culture medium of the invention is a defined medium, or a serum-free medium. For example, the compositions of the invention may be supplemented to the CHOMACS CD medium of Miltenyi Biotech (Bergisch Gladbach, Germany), to the PowerCHO-2 CD medium available from LONZA (Basel, Switzerland), the Acti-CHO P medium of PAA (PAA Laboratories, Pasching, Austria), the Ex-Cell CD CHO medium available from SAFC, the SFM4CHO medium and the CDM4CHO medium of ThermoFisher (Waltham, USA). The dipeptides of the invention may also be supplemented to DMEM medium (Life Technologies Corp., Carlsbad, USA). The invention, however, is not limited to supplementation of the above media.

In other preferred embodiments, the culture medium is a liquid medium in 2-fold, 3-fold, 3.33-fold, 4-fold, 5-fold or 10-fold concentrated form (volume/volume), relative to the concentration of said medium in use. This allows preparation of a "ready-to-use" culture medium by simple dilution of the concentrated medium with the respective volume of sterile water. Such concentrated forms of the medium of the invention may also be used by addition of the same to a culture, e.g., in a fed-batch cultivation or perfusion process.

The cell culture medium (cell or tissue culture basal, feed or perfusion medium) of the present invention may preferably contain all nutrients required for sustained growth and product formation. Recipes for preparing culture media, in particular cell culture media, are well known to the person skilled in the art (see, e.g., Cell Culture Technology for Pharmaceutical and Cell-Based Therapies, Öztürk and Wei-Shou Hu eds., Taylor and Francis Group 2006). Various culture media are commercially available from various sources.

The culture media of the invention may preferably include a carbohydrate source. The main carbohydrate used in cell culture media is glucose, routinely supplemented at 5 to 25 mM. In addition, any hexose, such as galactose, fructose, or mannose or a combination may be used.

The culture medium typically may also include at least the essential amino acids (i.e., His, Ile, Leu, Lys, Met, Phe, Thr, Try, Val) as well as non-essential amino acids. A non-essential amino acid is typically included in the cell culture medium if the cell line is not capable of synthesizing the amino acid or if the cell line cannot produce sufficient quantities of the amino acid to support maximal growth. In addition, mammalian cells can also use glutamine as a major energy source. Glutamine is often included at higher concentrations than other amino acids (2-8 mM). However, as noted above, glutamine can spontaneously break down to form ammonia and certain cell lines produce ammonia faster, which is toxic.

The culture media of the invention may preferably comprise salts. Salts are added to the cell culture medium to maintain isotonic conditions and prevent osmotic imbalances. The osmolality of a culture medium of the invention is about 300 mOsm/kg, although many cell lines can tolerate an approximately 10 percent variation of this value or higher. The osmolality of some insect cell cultures tend to be higher than 300 mOsm/kg, and this may be 0.5 percent, 1 percent, 2 to 5 percent, 5- 10 percent, 10-15 percent, 15- 20 percent, 20-25 percent, 25-30 percent higher than 300 mOsm/kg. The most commonly used salts in cell culture medium include Na⁺, K⁺, Mg²⁺, Ca²⁺, Cl⁻, SO₄²⁻, PO₄³⁻, and HCO₃⁻ (e.g., CaCl₂, KCl, NaCl, NaHCO₃, Na₂HPO₄).

Other inorganic elements may be present in the culture medium. They include Mn, Cu, Zn, Mo, Va, Se, Fe, Ca, Mg, Si, and Ni. Many of these elements are involved in enzymatic activity. They may be provided in the form of salts such as CaCl₂, Fe(NO₃)₃, MgCl₂, MgSO₄, MnCl₂, NaCl, NaHCO₃, Na₂HPO₄, and ions of the trace elements, such as, selenium, vanadium and zinc. These inorganic salts and trace elements may be obtained commercially, for example from Sigma (Saint Louis, Missouri).

The culture media of the invention preferably comprise vitamins. Vitamins are typically used by cells as cofactors. The vitamin requirements of each cell line vary greatly, although generally extra vitamins are needed if the cell culture medium contains little or no serum or if the cells are grown at high density. Exemplary vitamins preferably present in culture media of the invention include biotin, choline chloride, folic acid, i-inositol, nicotinamide, D-Ca⁺⁺-pantothenate, pyridoxal, riboflavin, thiamine, pyridoxine, niacinamide, A, B₆, B₁₂, C, D₃, E, K, and p-aminobenzoic acid (PABA).

Culture media of the invention may also comprise serum. Serum is the supernatant of clotted blood. Serum components include attachment factors, micronutrients (e.g., trace elements), growth factors (e.g., hormones, proteases), and protective elements (e.g., antitoxins, antioxidants, antiproteases). Serum is available from a variety of animal sources including human, bovine or equine serum. When included in cell culture medium according to the invention, serum is typically added at a concentration of 5-10 %(vol.). Preferred cell culture media are serum-free.

To promote cell growth in the absence or serum or in serum reduced media, one or more of the following polypeptides can be added to a cell culture medium of the invention: for example, fibroblast growth factor (FGF), including acidic FGF and basic FGF, insulin, insulin-like growth factor (IGF), epithelial growth factor (EGF), nerve growth factor (NGF), platelet-derived growth factor (PDGF), and transforming growth factor (TGF), including TGFalpha and TGFbeta, any cytokine, such as interleukins 1, 2, 6, granulocyte stimulating factor, leukocyte inhibitory factor (LIF), etc.

In other embodiments, the cell culture medium does not comprise polypeptides (i.e., peptides with more than 20 amino acids).

One or more lipids can also be added to a cell culture medium of the invention, such as linoleic acid, linolenic acid, arachidonic acid, palmitoleic acid, oleic acid, polyenoic acid, and/or fatty acids of 12, 14, 16, 18, 20, or 24 carbon atoms, each carbon atom branched or unbranched), phospholipids, lecithin (phophatidylcholine), and cholesterol. One or more of these lipids can be included as supplements in serum-free media. Phosphatidic acid and lysophosphatidic acid stimulate the growth of certain anchorage-dependent cells, such as MDCK, mouse epithelial, and other kidney cell lines, while phosphatidylcholine, phosphatidylethanolamine, and phosphatidylinositol stimulate the growth of human fibroblasts in serum-free media. Ethanolamine and cholesterol have also been shown to promote the growth of certain cell lines. In certain embodiment, the cell culture medium does not contain a lipid.

One or more carrier proteins, such as bovine serum albumin (BSA) or transferrin, can also be added to the cell culture medium. Carrier proteins can help in the transport of certain nutrients or trace elements. BSA is typically used as a carrier of lipids, such as linoleic and oleic acids, which are insoluble in aqueous solution. In addition, BSA can also serve as a carrier for certain metals, such as Fe, Cu, and Ni. In protein-free formulations, non-animal derived substitutes for BSA, such as cyclodextrin, can be used as lipid carriers.

One or more attachment proteins, such as fibronectin, laminin, and pronectin, can also be added to a cell culture medium to help promote the attachment of anchorage-dependent cells to a substrate.

The cell culture medium can optionally include one or more buffering agents. Suitable buffering agents include, but are not limited to, N-[2-hydroxyethyl]-piperazine- N'-[2-ethanesulfonic acid] (HEPES), MOPS, MES, phosphate, bicarbonate and other buffering agents suitable for use in cell culture applications. A suitable buffering agent is one that provides buffering capacity without substantial cytotoxicity to the cells cultured. The selection of suitable buffering agents is within the ambit of ordinary skill in the art of cell culture.

Polyanionic or polycationic compounds may be added to the culture medium to prevent the cells from clumping and to promote growth of the cells in suspension.

In a preferred embodiment, the culture medium is in liquid form. The culture medium, however, can also be a solid medium, such as a gel-like medium, e.g. an agar-agar-, carrageen- or gelatine-containing medium (powders, aggregated powders, instantized powders etc.). Preferably, the culture medium is in sterile form.

The culture medium of the present invention can be in concentrated form. It may be, e.g., in 2- to 100-fold concentrated form, preferably in 2-fold, 3-fold, 3.33-fold, 4-fold, 5-fold, 10-fold, 20-fold, 50-fold or 100-fold (relative to a concentration that supports growth and product formation of the cells).

Such concentrated culture media are helpful for preparing the culture medium for use by dilution of the concentrated culture medium with an aqueous solvent, such as water. Such concentrated culture media may be used in batch culture but are also advantageously used in fed-batch or continuous cultures, in which a concentrated nutrient composition is added to an ongoing cultivation of cells, e.g., to replenish nutrients consumed by the cells during culture.

In other embodiments of the invention, the culture medium is in dry form, e.g., in form of a dry powder, or in form of granules, or in form of pellets, or in form of tablets.

The present invention also relates to the use of a culture medium of the invention for culturing cells. Another aspect of the invention relates to the use of a culture medium of the invention for producing a cell culture product.

A preferred embodiment of the invention relates to the use of a culture medium according to the invention for culturing animal cells or plant cells, most preferred mammalian cells. In specific embodiments the cells to be cultured are CHO cells, COS cells, VERO cells, BHK cells, HEK cells, HELA cells, AE-1 cells, insect cells, fibroblast cells, muscle cells, nerve cells, stem cells, skin cells, endothelial cells and hybridoma cells. Preferred cells of the invention are CHO cells and hybridoma cells. Most preferred cells of the invention are CHO cells. Particularly preferred CHO cells of the invention are CHO DG44 and CHO DP12 cells.

Also included in the scope of the present invention is a method of culturing cells, said method comprising contacting said cells with a cell culture medium according to the invention. In one embodiment of the invention, the method of culturing cells comprises contacting the cell with a basal culture medium under conditions supporting the cultivation of the cell and supplementing the basal cell culture medium with a concentrated medium according to the present invention. In preferred embodiments, the basal culture medium is supplemented with the concentrated feed or medium on more than one day.

Another aspect of the invention relates to a method of producing a culture medium according to the invention, wherein said culture medium comprises a composition according to the invention. Methods of producing a culture medium according to the invention comprise at least one step of adding the composition of the invention to the culture medium. Likewise, an aspect of the invention relates to the use of a composition of the invention for producing a cell culture medium.

Another aspect of the invention relates to a method of modifying a culture medium, wherein said modifying of said culture medium comprises addition of the composition of the invention to said culture medium.

Another aspect of the invention relates to a method of producing a liquid culture medium, said method comprising providing solid medium according to the invention, e.g., in form of a dry powder, or in form of granules, or in form of pellets, or in form of tablets; and dissolving said solid culture medium in an aqueous medium, such as water.

Another aspect of the invention relates to the use of a composition according to the invention in a culture medium for culturing cells. Another aspect of the invention relates to the use of a composition according to the invention for cell culture.

The invention also relates to methods of manufacturing a cell culture product comprising the steps of (i) providing a cell capable of producing said cell culture product; (ii) contacting said cell with a culture medium of the invention; and (iii) obtaining said cell culture product from said culture medium or from said cell. Likewise, the present invention relates to the use of a composition according to the invention for manufacturing a cell culture product.

In preferred methods, the cell culture product is a therapeutic protein, a diagnostic protein, a polysaccharide, such as heparin, an antibody, a monoclonal antibody, a growth factor, an interleukin, virus, virus-like particle or an enzyme

Cultivation of cells, according to the invention can be performed in batch culture, in fed-batch culture or in continuous culture.

### Examples

### Materials:

**Table 1: Materials used for in vitro Glutathione assay**

| **Material** | **Supplier** |
|---|---|
| CHO-K1 (hamster ovary cells) | DSMZ, Braunschweig (Germany) |
| Ham's F-12 Medium | Lonza, Basel (Switzerland) |
| Gentamicin (50 mg/ml) | Thermo Fisher Scientific, Massachusetts (USA) |
| Fetal bovine serum | Thermo Fisher Scientific, Massachusetts (USA) |
| Pipet tips | Biozym, Hessisch Oldendorf (Germany) |
| 96 well plate, white, flat bottom | Greiner Bio-One, Kremsmünster (Austria) |
| Falcon tubes | Greiner Bio-One, Kremsmünster (Austria) |
| CombiTips | Eppendorf, Hamburg (Germany) |
| L-Cysteine | Sigma-Aldrich, Missouri (USA) |
| N,N-di-L-alanyl-L-cystine | Evonik Operations GmbH, Darmstadt (Germany) |
| (Lys-Cys)2 | Evonik Operations GmbH, Darmstadt (Germany) |
| 3% Hydrogen peroxide solution | Sigma-Aldrich, Missouri (USA) |
| GSH/GSSG-Glo^{™} Assay Kit | Promega GmbH, Madison (USA) |

**Table 2: Devices used for Glutathione assay.**

| **Device** | **Supplier** |
|---|---|
| Safety cabinet HERA Safe 2020 | Thermo Fisher Scientific GmbH, Dreieich (Germany) |
| Heracell^{™} 150i CO2 Incubator | Thermo Fisher Scientific GmbH, Dreieich (Germany) |
| Automatic cell counter Countess | Thermo Fisher Scientific GmbH, Dreieich (Germany) |
| Centrifuge 5415R | Eppendorf, Hamburg (Germany) |
| Vortex- Genie 2 | Scientific Industries, Bohemia (USA) |
| TECAN Infinite 200 Pro | Tecan Group Ltd., Männedorf (Switzerland) |
| Microscope Primo Vert | Carl Zeiss AG |
| Analytical Balance | Sartorius AG |
| Eppendorf Pipets | Eppendorf, Hamburg (Germany) |
| Dispenser | Eppendorf, Hamburg (Germany) |

**Table 3: Materials used for in vitro cytotoxicity assay**

| **Material** | **Supplier** |
|---|---|
| Human bone marrow stromal cells, (Cat.No 70071) | Stemcell, Vancouver (Canada) |
| MesenCult^{™}-ACF Plus Culture Kit | Stemcell, Vancouver (Canada) |
| Gentamicin (50 mg/ml) | Thermo Fisher Scientific, Massachusetts (USA) |
| Glutamine | Stemcell, Vancouver (Canada) |
| L-Cysteine | Sigma-Aldrich, Missouri (USA) |
| (N,N'-di-L-alanyl-L-cystine), (Ala-Cys)₂ | Evonik Operations GmbH, Darmstadt (Germany) |
| Cell culture microplate, 96 well, white | Greiner Bio-One, Kremsmünster (Austria) |
| CellTiter-Glo Luminescent Cell Viability Assay, (Cat. No G7570) | Promega GmbH, Madison (USA) |

**Table 4: Devices used for cytokine release assay.**

| **Device** | **Supplier** |
|---|---|
| Safety cabinet HERA Safe 2020 | Thermo Fisher Scientific GmbH, Dreieich (Germany) |
| Heracell^{™} 150i CO2 Incubator | Thermo Fisher Scientific GmbH, Dreieich (Germany) |
| Automatic cell counter Countess | Thermo Fisher Scientific GmbH, Dreieich (Germany) |
| Centrifuge 5415R | Eppendorf, Hamburg (Germany) |
| Vortex- Genie 2 | Scientific Industries, Bohemia (USA) |
| TECAN Infinite 200 Pro | Tecan Group Ltd., Männedorf (Switzerland) |

### Methods:

### In vitro Glutathione assay in Chinese hamster ovary cells (subclone -K1):

The addition of Cys-peptides and mixtures of Cys-peptides with L-cysteine and L-cystine to the cell culture medium was evaluated regarding their influence on the intracellular glutathione level. Oxidative stress was simulated by the addition of hydrogen peroxide solution.

The assay was performed with Chinese hamster ovary cells (CHO-K1). The cells were seeded in a white 96-well cell culture plate at a cell density of 10.000 cells/well in a volume of 50µl/well. In relation to the oxidative stress conditions the cells were pre-incubated or constantly incubated with Cys-peptide samples. Therefor two-fold concentrated samples were added directly after seeding in a volume of 50 µl/well to achieve a 1:2 dilution.

The samples (Ala-Cys)₂ and (Lys-Cys)₂ were dissolved in culture medium, either containing 10% fetal bovine serum (FBS) or medium without FBS, to perform the assay setup in presence and absence of FBS during the stress phase. Each sample was tested in triplicates and a background control (without cells) for each sample was used. Dipeptides were tested in a concentration of 2 mM and in mixtures with L-cysteine and L-cystine in a final concentration of 0.1 mM. L-Cysteine alone was also tested at concentrations of 2 mM and 0.5 mM. Medium without sample was added to control experiments to maintain otherwise comparable conditions and to determine the effect of the culture medium during oxidative stress conditions.

An additional medium control for each assay condition (with and without FBS), but not stressed with H₂O₂, was used as additional control to determine the effect of H₂O₂ addition to the test system.

After an incubation time of 24 hours, supernatants from pre-incubated wells were changed to culture medium in a volume of 50µl/well. In wells with constantly incubated samples, the supernatants were exchanged to fresh medium still containing Cys-peptide samples in a volume of 50 µl/well.

Then a 200 µM hydrogen peroxide (H₂O₂) solution was added to the cells in a volume of 50 µl/well to start the stress phase. The final H₂O₂ concentration in the assay was 100 µM in a final volume of 100 µl/well. Incubation of cells with H₂O₂ took place for 20 min. at 37 °C and 5% CO₂.

After the incubation time with H₂O₂, the supernatants were discarded completely and the GSH/GSSG assay was performed according to the manufacturer's protocol to measure the total intracellular Glutathione (GSH) level.

Total glutathione lysis reagent was added to the cells in a volume of 50 µl/well. Plate was gently shaken on an orbital shaker for 5 minutes at room temperature. Luciferin generation reagent was added with 50 µl/well and plate was shaken again, followed by an incubation time of 30 minutes at room temperature. After incubation time, luciferin detection reagent was added in a volume of 100 µl/well. After a further shaking step, the covered plate was incubated at room temperature for 15 min.

As a result of enzyme reactions in the different reagents, luciferin is formed proportional to the glutathione concentration in the cells. This leads to luminescence signals which were measured in a multiplate reader.

To calculate the GSH concentrations, a dilution series of a glutathione standard was performed, and luminescence signals measured. Relative luminescence signals were plotted against GSH concentrations. After subtracting the background signals, GSH concentration in samples was calculated based on the linear standard curve.

### In vitro cytotoxicity assay on mesenchymal stem cells:

The assay was performed with human bone marrow stromal cells, mesenchymal stem cells (MSC). In a first step, the cells were seeded in a white 96-well cell culture plate and incubated for 24 h in a CO₂-Incubator (37°C, 5% CO₂, 95% humidity) at a cell density of 5.000 cells/well. After the resting time of 24 hours, diluted samples containing test compounds were added to the plate in equal volumes of 50 µl to reach the desired concentrations of interest and a final volume of 100 µl per well.

Samples of L-cysteine were compared to Cys-peptides and each sample was tested in triplicates.

The control was rested in medium without sample. After the resting period of 24 hours in a CO₂-Incubator (37°C, 5% CO₂, 95% humidity) the assay reagent was constituted according to the manufacturers manual and the reagent was added to the well in a volume of 100 µl. The viability was determined based on the quantification of the ATP present in the cells per well. Through the luminescence detection of luciferase using a multiplate reader the ATP amount was measured and quantified. Resulting luminescence signal of samples was normalized to the signal of control cells.

### Example 1: Effects of Cys-peptides and mixtures on intracellular glutathione level after induced oxidative stress

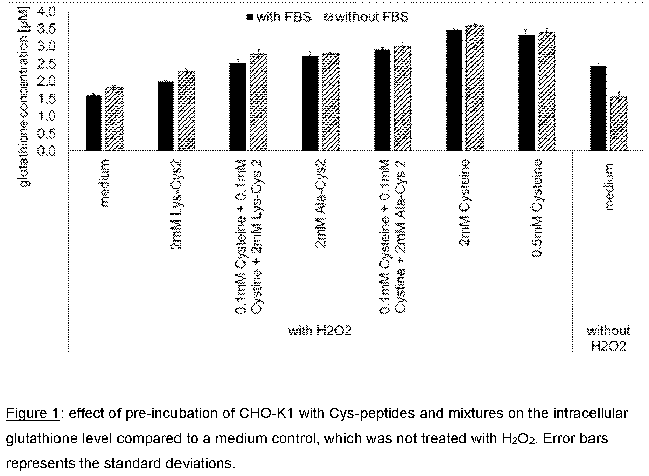

CHO-K1 cells were cultured and either pre-incubated for 24 hours or constantly incubated with Cys-peptide samples for 24 hours as well as during oxidative stress phase. These conditions were tested with and without FBS in culture medium. After luminescence signals were measured, the intracellular glutathione concentration of cells incubated with Cys-peptides and mixtures and treated with 100 µM H₂O₂ were compared to a medium control, which was not treated with H₂O₂. The GSH concentrations of CHO-K1 cells pre-incubated with the samples are shown in Figure 1.

Figure 1 shows the effect of pre-incubation of CHO-K1 with Cys-peptides and mixtures on the intracellular glutathione level compared to a medium control, which was not treated with H2O2. Error bars represents the standard deviations.

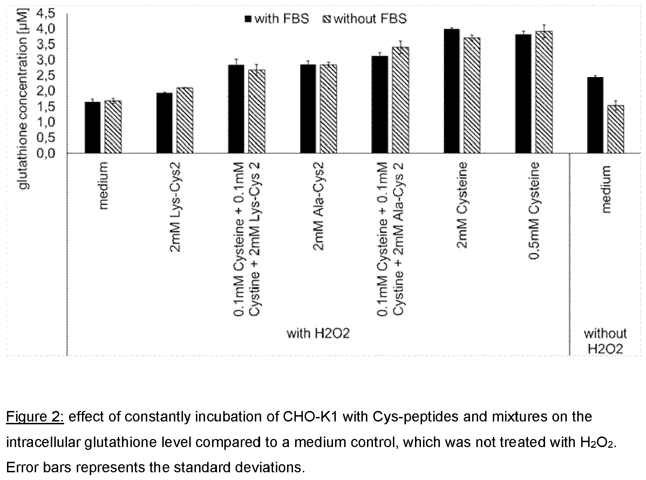

The GSH concentrations of CHO-K1 cells constantly incubated with samples are shown in Figure 2.

Figure 2 shows the effect of constantly incubation of CHO-K1 with Cys-peptides and mixtures on the intracellular glutathione level compared to a medium control, which was not treated with H2O2. Error bars represents the standard deviations.

It was shown that incubation with Cys-peptides and mixtures not only maintains the intracellular glutathione level in the context of oxidative stress, but also increases it in CHO-K1 cells. Furthermore, the constant incubation showed an even higher glutathione concentration than the pre-incubation. This effect could be shown in serum-containing as well as serum-free media conditions.

Incubation with Cysteine showed a similar effect, but in comparison to the Cys-peptides or mixtures the high reactivity of cysteine to almost insoluble cystine in media could mediate further cultivation issues such as precipitation. Carta et al. 1996, J. Chem. Eng. Data, 41, 414-417 investigated cystine solubility as a function of pH and found that the solubility is only around 1 mM at cell culture media relevant pH of about 7. This limits the formulation of concentrated, pH neutral feeds that can contribute to sustaining the glutathione level.

Therefore, a method to increase intracellular glutathione in cultured cells through addition of Cys-peptides and mixtures without further negative side effects such as precipitation, could be identified.

### Example 2: The solubility and biocompatibility of Cys-peptides is higher than that of L-cystine and L-cysteine, respectively

While L-cysteine was shown to increase intracellular glutathione concentration as well, it is difficult to supply enough of it in cell culture media and processes because it rapidly oxidizes to L-cystine in the presence of oxygen or other oxidants. In the reduced form it can become toxic or inhibitory.

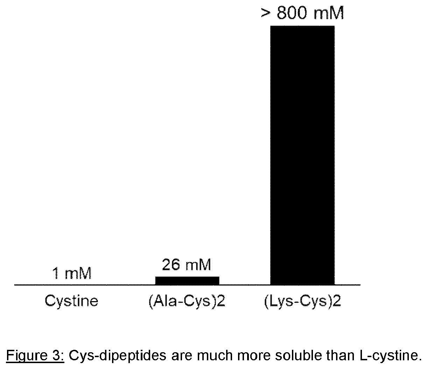

To determine solubility of L-cystine and Cys-peptides, increasing amounts of the respective products were dissolved at 25 °C in a beaker with 0,1 M phosphate buffer (pH = 7) under magnetic stirring until the solubility limit was reached. The pH was continuously monitored with a pH electrode and if necessary readjusted to pH = 7 with NaOH. Measured maximum solubility of the different components is shown in Figure 3. The solubility of the Cys-peptides is manyfold higher than that of L-cystine.

The toxic or inhibitory effects of L-cysteine were compared to Cys-peptides by conducting viability assays with a commercial kit (CellTiter-Glo^{®} Luminescent Cell Viability Assay, Promega)

The assay was performed with human bone marrow stromal cells, mesenchymal stem cells (MSC). In a first step, the cells were seeded in a white 96-well cell culture plate and incubated for 24 h in a CO₂-Incubator (37°C, 5% CO₂, 95% humidity) at a cell density of 5.000 cells/well. After the resting time of 24 hours, samples containing test compounds dissolved in medium were added to the plate in equal volumes of 50 µl to reach the desired concentrations of interest and a final volume of 100 µl per well.

Different concentrations of L-cysteine and (Ala-Cys)₂ were tested. Each sample was tested in triplicates.

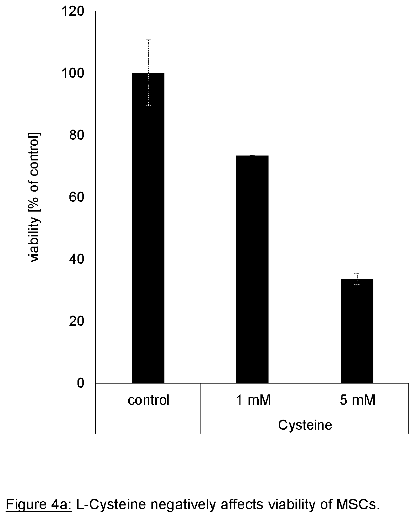

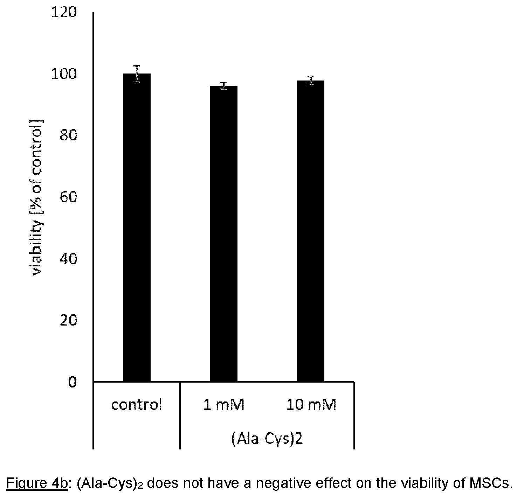

For controls, only medium without addition of sample was tested. After the resting period of 24 hours in a CO₂-Incubator (37°C, 5% CO₂, 95% humidity) the assay reagent was constituted according to the manufacturers manual and the reagent was added to the well in a volume of 100 µl. The viability was determined based on the quantification of the ATP present in the cells per well. Through the luminescence detection of luciferase using a multiplate reader the ATP amount was measured and quantified. Resulting luminescence signal of samples was normalized to the signal of control cells. The results are shown in Figure 4a and Figure 4b.

Figure 4a shows that L-Cysteine negatively affects viability of MSCs and Figure 4b shows that (Ala-Cys)₂ does not have a negative effect on the viability of MSCs.

## Claims

1. A method for increasing intracellular level of glutathione in cultured cells, wherein the cultured cells are growing in a cell culture medium, wherein the cell culture medium comprises at least one oligopeptide of 2-10 amino acids in length, said amino acids being natural amino acids and at least one of said amino acid being cysteine (Cys).

2. The method according to claim 1, wherein the cell culture medium further comprises free cysteine.

3. The method according to any of the preceding claims, wherein a molar ratio of the oligopeptide bound cysteine to the free cysteine in the cell culture medium is from about 0.1 to 10, preferably from about 0.2 to 4, most preferable from about 0.5 to 2.

4. The method according to any of the preceding claims, wherein the oligopeptide is a dipeptide and is Xxx-Cys or Cys-Xxx, wherein Xxx is a natural amino acid, the dipeptide preferably being Asp-Cys, Cys-Asp, Lys-Cys, Cys-Lys, Ala-Cys or Pro-Cys, most preferable Ala-Cys, Cys-Ala, Lys-Cys or Cys-Lys.

5. The method according to any one of the preceding claims, wherein cysteine is added to an aqueous solution at a concentration of at least 1mM, preferably at least 10 mM, more preferably at least 25 mM, most preferably at least 50 mM.

6. Use of a cell culture medium for increasing intracellular level of glutathione in cultured cells, preferably as an aqueous stock or feed solution,
wherein the cell culture medium comprises at least one oligopeptide of 2-10 amino acids in length, said amino acids being natural amino acids and at least one of said amino acid being cysteine (Cys) and free cysteine.

7. Use of claim 6, wherein said cells are selected from the list consisting of CHO cells, COS cells, VERO cells, BHK cells, HEK cells, HELA cells, AE-1 cells, insect cells, fibroblast cells, muscle cells, nerve cells, stem cells, skin cells, endothelial cells, immune cells such as NK or T-cells and hybridoma cells.
